Europäisches Patentamt

European Patent Office

Office européen des brevets

(19)

(11) EP 0 867 171 A2

(12) EUROPÄISCHE PATENTANMELDUNG

(43) Veröffentlichungstag:
30.09.1998 Patentblatt 1998/40

(51) Int. Cl.6: A61K 7/13

(21) Anmeldenummer: 98103188.3

(22) Anmeldetag: 24.02.1998

(84) Benannte Vertragsstaaten:
AT BE CH DE DK ES FI FR GB GR IE IT LI LU MC
NL PT SE
Benannte Erstreckungsstaaten:
AL LT LV MK RO SI

(30) Priorität: 27.03.1997 DE 19712860

(71) Anmelder:
Wella Aktiengesellschaft
64274 Darmstadt (DE)

(72) Erfinder:
• Kunz, Manuela, Dr.
  1723 Marly (CH)
• Le Cruer, Dominique
  1723 Marly (CH)
• Irrgang, Bernhard, Dr.
  1713 St. Anton (CH)

(54) **Pulverförmiges Mittel zum Herstellen von Gelen, enthaltend ein Methylhydroxyalkylcellulose**

(57) Es wird ein pulverförmiges Mittel zur Herstellung von kosmetisch verwendbaren Gelen beschrieben, das Methylhydroxyalkylcellulose, insbesondere eine hochverätherte Methylhydroxyethylcellulose, welche in einer 2prozentigen wäßrigen Lösung eine Viskosität von 7.000 bis 12.000 mPas aufweist, enthält.

**Beschreibung**

Gegenstand der Erfindung ist ein pulverförmiges Mittel zum Herstellen von kosmetisch verwendbaren Gelen, das mit Wasser versetzt nach wenigen Minuten Schütteln ein gebrauchsfertiges Gel ergibt.

Gele sind formbeständige, leicht deformierbare disperse Systeme aus mindestens zwei Komponenten. Die eine Komponente besteht meistens aus einem festen, kolloidal zerteilten Stoff mit langen oder stark verzweigten Teilchen wie Cellulosether, Gelatine, Kieselsäure, Polysaccharide, Pectine u.a., während es sich bei der anderen Komponente um eine Flüssigkeit, im allgemeinen Wasser, als Dispersionsmittel handelt. Der Gelbildner formt in der Flüssigkeit ein räumliches Netzwerk. Sind die Hohlräume zwischen den Teilchen mit Flüssigkeit gefüllt, so spricht man von Lyogelen, sind sie mit Wasser gefüllt, von Hydrogelen.

Pulvermischungen, die mit Wasser erst vor der Anwendung in ein gebrauchsfertiges Produkt, z. B. ein Gel, überführt werden, sind schon seit langem bekannt. Zum Beispiel sind oxidative Haarfärbepulver im Handel, die in Wasser geschüttet und gerührt werden und dann ein zur Haarfärbung einsetzbares Gel bilden. Die Darreichung in Pulverform hat neben der Gewichtseinsparung gegenüber dem Fertigprodukt, die bei Reisen oder generell bei den Transportkosten eine Rolle spielt, den Vorteil, daß man wenig lager-stabile, z. B. feuchtigkeitsempfindliche Rohstoffe wie Enzyme verwenden kann.

Das eigentliche Problem bei der Herstellung eines gebrauchsfertigen, kosmetisch einsetzbaren Gels aus einer pulverförmig vorliegenden Mischung, die einen Gelbildner enthält, besteht darin, daß es bisher nicht möglich war, Gelbilder zu finden, die nach Zugabe eines Lösungsmittels in wenigen Augenblicken ein homogenes Gel zu bilden vermögen. Die bekannten, zur Gelbildung eingesetzten Trockensubstanzen erfordern ein Erhitzen und/oder intensives Rühren oder Schütteln über einen längeren Zeitraum. Trotzdem lassen sich dabei ungelöste Reste des Verdickungsmittels nicht vermeiden. Hinzu kommt, daß einige Gelbildner nur in einem begrenzten pH-Bereich einsetzbar sind. Die schnelle, unkomplizierte Überführung einer als Pulver vorliegenden Mischung in ein kosmetisch einsetzbares Gel stellt daher nach wie vor ein großes Problem dar.

Erfindungsgemäß wird diese Aufgabe durch ein pulverförmiges Mittel gelöst, das eine Methylhydroxyalkylcellulose, welche in einer 2prozentigen wäßrigen Lösung eine Viskosität von 7.000 bis 12.000 mPa·s (gemessen mit einem Kugelfallviskosimeter nach Höppler bei T= 20°C) aufweist , enthält. Bevorzugt wird hierbei Methylhydroxyethylcellulose, insbesondere eine hochveretherte Methylhydroxyethylcellulose, die in einer 2prozentigen wäßrigen Lösung eine Viskosität von etwa 10.000 mPa·s aufweist. Der Veretherungsgrad sollte zwischen 1,5 und 2,0, vorzugsweise bei 1,8, liegen, während das Molekulargewicht gleich 200.000 bis 250.000, vorzugsweise 225.000, sein sollte. Ein derartiges Cellulosederivat wird beispielsweise von der Firma Hoechst unter dem Handelsnamen Tylose® MHB 10.000 P verkauft.

Im allgemeinen ist die Methylhydroxyalkylcellulose in dem erfindungsgemäßen pulverförmigen Mittel zum Herstellen von kosmetisch verwendbaren Gelen in einer Menge von etwa 0,5 bis 50 Gewichtsprozent, vorzugsweise 1 bis 10 Gewichtsprozent, enthalten.

Bei den erfindungsgemäß einzusetzenden Methylhydroxyalkylcellulosen handelt es sich um hochhydrophile Verbindungen, die bei längerer Lagerzeit zur Feuchtigkeitsaufnahme und damit zum Verklumpen neigen. Dies kann jedoch verhindert werden, wenn dem erfindungsgemäßen Mittel Füllstoffe und/oder Trocknungsmittel, wie zum Beispiel festes, hochdisperses Kieselgel und/oder Magnesiumsulfat, zugesetzt werden.

Zur Einstellung eines geeigneten pH-Wertes können feste, alkalisierende Mittel, wie zum Beispiel Alkalihydroxide, Alkalicarbonate oder Ammoniumcarbonate, oder feste Säuren, wie zum Beispiel Zitronensäure, Glykolsäure, Ascorbinsäure oder Borsäure, zugesetzt werden.

Das erfindungsgemäße pulverförmige Mittel zum Herstellen von kosmetisch verwendbaren Gelen kann außerdem alle die Inhaltsstoffe enthalten, die in einem Tönungsgel, einem Haarfärbegel, einem Mittel zur Entfärbung von Fasern oder einem Shampoo üblicherweise enthalten sind.

Zur Herstellung von Tönungsmitteln kann das erfindungsgemäße Mittel die folgenden Nitrofarbstoffe, Chinonfarbstoffe, basischen oder sauren Farbstoffe oder neutralen Azofarbstoffe enthalten, sofern sie in fester Form vorliegen und wasserlöslich sind.

**Nitrofarbstoffe**

1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6- nitrobenzol (HC Violet No. 1), 4-[Ethyl-(2-hydroxyethyl)amino]-1-[(2-hydroxyethyl)amino]-2- nitrobenzol-hydrochlorid (HC Blue No. 12), 4-[Di(2-hydroxyethyl)amino]-1-[(2-methoxyethyl)-amino]-2-nitrobenzol HC Blue No. 11), 1-[(2,3- Dihydroxy-propyl)amino]-4-[methyl-(2-hydroxyethyl)-amino]- 2-nitrobenzol (HC Blue No. 10), 1-[(2,3-Dihydroxypropyl)amino]-4-methyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)- amino]-2-nitrobenzol (HC Blue No. 6), 2-((4-Amino-2-nitrophe-

2

nyl)amino)- 5-dimethylamino-benzoesäure (HC Blue No. 13), 1-Amino- 4-[(2-hydroxyethyl)amino]-2-nitrobenzol (HC Red No. 7), 2- Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxy- ethyl)amino]-2-nitrobenzol, 4-Amino-1-[(2-hydroxyethyl) amino]-2-nitrobenzol (HC Red No. 3), 4-Amino-3-nitrophenol, 4-[(2-Hydroxyethyl)amino]-3-nitrophenol, 1-[(2-Aminoethyl) amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-hlor-4-[(2,3-dihydroxypropyl)amino]-2-nitrobenzol (HC Red No. 10), 5-Chlor-1,4- [di(2,3-dihydroxypropyl)amino] - 2-nitrobenzol (HC Red No.11), 2-[(2-Hydroxyethyl)amino]-4,6-dinitro-phenol, 4-Ethylamino- 3-nitrobenzoesäure, 2-[(4-Amino-2-nitrophenyl) amino]-benzoesäure, 2-Chlor-6-ethylamino-4-nitrophenol, 2-Amino-6-chlor-4-nitrophenol,4- [(3-Hydroxypropyl) amino]-3-nitrophenol, 2,5-Diamino-6-nitropyridin, 1,2,3,4-Tetrahydro-6- nitrochinoxalin, 7-Amino-3,4-dihydro-6-nitro-2H-1,4-benzoxazin (HC Red No. 14), 1-Amino-2-[(2-hydroxyethyl)amino]-5-nitro- benzol (HC Yellow No. 5), 1-(2-Hydroxyethoxy)-2-[(2-hydroxyethyl)amino]-5-nitrobenzol (HC Yellow No. 4), 1-[(2- Hydroxyethyl)amino]-2-nitrobenzol (HC Yellow No. 2), 2-[(2- Hydroxyethyl)amino]-1-methoxy-5-nitrobenzol, 2-Amino-3- nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl) amino]-5-nitrobenzol (HC Yellow No. 10), 4-[(2-Hydroxyethyl) amino]-3-nitro-1-methylbenzol, 1-Chlor-4-[(2-hydroxyethyl)amino]-3-nitrobenzol (HC Yellow No. 12), 4-[(2-Hydroxyethyl) amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15).

## Chinonfarbstoffe

1,4-Di[(2,3-dihydroxypropyl)amino]-9,10-anthrachinon, 1-[(2-Hydroxyethyl)amino]-4-methylamino-9,10-anthrachinon (CI61505, Disperse Blue No. 3), 2-[(2-Aminoethyl)amino]-9,10-anthrachinon, 1-Hydroxy-4-[(4-methyl-2-sulfophenyl)amino]-9,10-anthrachinon, 1-[(3-Aminopropyl)amino]-4-methylamino- 9,10-anthrachinon, 1-[(3-Aminopropyl)amino] - 9,10-anthrachinon, 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69).

## Basische Farbstoffe

9-(Dimethylamino)-benzo[a]phenoxazin-7-ium-chlorid (CI51175; Basic Blue No. 6), Di[4-(diethylamino) phenyl][4-(ethylamino)-naphthyl]carbenium-chlorid (CI42595; Basic Blue No. 7), 3,7-Di(dimethylamino)phenothiazin-5-ium-chlorid (CI52015; Basic Blue No. 9), Di[4-(dimethylamino)phenyl]-[4-(phenylamino)naphthyl]carbenium-chlorid (CI44045; Basic Blue No. 26), 2-[(4-(Ethyl(2-hydroxyethyl) amino) phenyl) azo]-6- methoxy-3-methylbenzothiazolium-methylsulfat (CI11154; Basic Blue No. 41), 8-Amino-2-brom-5-hydroxy- 4-imino-6- [(3- (trimethylammonio)phenyl)amino]-1(4H)-naphthalinon-chlorid (CI56059; Basic Blue No. 99), Bis[4-(dimethylamino) phenyl] [4-(methylamino)phenyl]carbenium-chlorid (CI42535; Basic Violet No. 1), Tris[4-(dimethylamino)phenyl] carbenium-chlorid (CI42555; Basic Violet No. 3), 2-[3,6-(Diethylamino)dibenzopyranium- 9-yl]-benzoesäure-chlorid(CI45170; Basic Violet No. 10), Di(4-aminophenyl) (4-amino-3-methylphenyl) carbenium-chlorid (CI42510; Basic Violet No. 14), 1,3-Bis-[(2,4-diamino-5-methylphenyl)azo]-3-methylbenzol (CI21010;Basic Brown No. 4), 1-[(4- Aminophenyl) azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (CI12251; Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl- 5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4- Dimethoxyphenyl)amino)ethenyl]-1,3,3-trimethyl-3H-indol-1-ium-chlorid (CI48055; Basic Yellow No. 11), 3-Methyl-1- phenyl-4- [(3-(trimethylammonio)phenyl)azo]-pyrazol-5-on-chlorid (CI12719; Basic Yellow No. 57), Bis[4-(diethylamino)phenyl] phenylcarbenium-hydrogensulfat (1:1) (CI42040; Basic Green No. 1).

## Neutrale Azofarbstoffe

1-[Di(2-hydroxyethyl)amino]-3-methyl-4-[(4-nitrophenyl)azo]-benzol (CI11210, Disperse Red No. 17), 4-[(4-Aminophenyl) azo]-1-[di(2-hydroxyethyl)amino]-3-methylbenzol (HC Yellow No. 7), 2,6-Diamino-3-[(pyridin-3-yl)azo]-pyridin.

## Saure Farbstoffe

6-Hydroxy-5-[(4-sulfophenyl)azo]-2-naphthalinsulfonsäure-dinatriumsalz (CI15985; Food Yellow No. 3; FD&G Yellow No. 6), 2,4-Dinitro-1-naphthol -7-sulfonsäure-dinatriumsalz (CI10316; Acid Yellow No. 1; Food Yellow No. 1), 2-(Indan- 1,3- dion - 2-yl)chinolin-x,x-sulfonsäure (Gemisch aus Mono- und Disulfonsäure) (CI47005;D&C Yellow No. 10; Food Yellow No. 13; Acid Yellow No. 3), 5-Hydroxy-1- (4- sulfophenyl)- 4-[(4-sulfophenyl)azo]-pyrazol-3-carbonsäure-trinatriumsalz (CI19140; Food Yellow No. 4; Acid Yellow No. 23), 9-(2-Carboxyphenyl)-6- hydroxy-3H-xanthen-3-on (CI45350; Acid Yellow No. 73; D&C Yellow No. 8), 5-[(2,4-Dinitrophenyl) amino]-2-phenylamino-benzolsulfonsäure-natriumsalz (CI10385; Acid Orange No. 3), 4-[(2,4-Dihydroxyphenyl) azo]- benzolsulfonsäure-mononatriumsalz (CI14270; Acid Orange No. 6), 4-[(2-Hydroxynaphth-1-yl)-azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 6-Hydroxy-5-[(4-sulfonaphth-1-yl)-azo]-2,4-naphthalin-disulfonsäure-trinatriumsalz (CI16255; Ponceau 4R; Acid Red No. 18), 3-Hydroxy-4-[(4-sulfonaphth-1-yl) azo]- 2,7- naphthalin-disulfonsäure-trinatriumsalz (CI16185; Acid Red No. 27), 8-Amino-1-hydroxy-2-(phenylazo)- 3,6- naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)- 9- (2,4-disulfophenyl)- 3H- xanthen- 3- yliden]-N-ethylethanaminium-hydroxid, inneres Salz, Natriumsalz (CI45100; Acid Red No. 52), 8-[(4-(Phenylazo)phenyl) azo]-7-naphthol-1,3-disulfonsäure-dinatriumsalz (CI27290; Acid Red No. 73), 2',4',5',7'-Tetrabrom- 3',6'-dihydroxyspiro [isobenzofuran-1(3H),9'-[9H]xanthen]-3-on-dinatriumsalz (CI45380; Acid Red No. 87), 2',4',5',7'- Tetrabrom-4,5,6,7-tetrachlor-3',6'-dihydroxyspiro-[isobenzofuran-1(3H),9'[9H]xanthen]-3-on-dinatriumsalz (CI45410; Acid Red No. 92), 3',6'-Dihydroxy-4',5'-diiodospiro-[isobenzofuran-1(3H),9'(9H)-xanthen)-3-on-dinatriumsalz (CI45425; Acid Red No. 95), (2-Sulfophenyl)di [4-(ethyl((4-sulfophenyl)methyl)amino)phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino) phenyl]-(3,7-disulfo-2-hydroxynaphth- 1-yl)-carbenium-inneres Salz, Mononatriumsalz (CI44090; Food Green No. 4; Acid Green No. 50), Bis[4-(diethylamino)phenyl] (2,4-disulfophenyl)carbenium, inneres Salz, Natriumsalz (2:1) (CI42045; Food Blue No. 3; Acid Blue No. 1), Bis[4-(diethylamino) phenyl](5-hydroxy-2,4- disulfophenyl)carbenium, inneres Salz, Calciumsalz (2:1) (CI42051; Acid Blue No. 3), 1-Amino-4-(cyclohexylamino)-9,10- anthrachinon-2-sulfonsäure-Natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, Mononatriumsalz (CI45190; Acid Violet No. 9), 1-Hydroxy-4-[(4-methyl-2-sulfophenyl) amino]- 9,10-anthrachinon-Natriumsalz (CI60730; D&C Violett No. 2; Acid Violet No. 43), Bisf3-nitro-4-[(4-phenylamino)- 3-sulfo-phenylamino]-phenyl[2]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2- hydroxynaphth- 1-yl)azo]-7-nitro-1-naphthalin-sulfonsäure-chromkomplex (3:2) (CI15711; Acid Black No. 52), 3-[(2,4-Dimethyl-5- sulfophenyl)azo]-4-hydroxy-1-naphthalinsulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]-naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1- sulfonsäure-Natriumsalz , Chrom-Komplex (Acid Red No. 195).

Der Gesamtgehalt an Farbstoffen beträgt dabei in dem erfindungsgemäßen Mittel vorzugsweise 0,01 bis 5 Gewichtsprozent.

Die Anwendung von zum Haarfärben geeigneten Oxidationsfarbstoffen mit Hilfe des erfindungsgemäßen pulverförmigen Mittels erfolgt dadurch, daß der erfindungsgemäßen Zubereitung die folgenden, als Entwickler und Kuppler bezeichnete Oxidationsfarbstoff-Vorstufen, im allgemeinen aromatische Amine und Phenole, zusammen mit festen Oxidationsmitteln wie Wasserstoffperoxid-Additionsprodukten oder insbesondere Enzymen, beigemischt werden.

## Entwickler

1,4-Diamino-benzol (p-Phenylendiamin), 1,4-Diamino-2- methyl-benzol (p-Toluylendiamin), 1,4-Diamino-2,6-dimethyl-benzol, 1,4-Diamino-2,5-dimethyl-benzol, 1,4-Diamino-2,3-dimethyl- benzol, 2-Chlor-1,4-diaminobenzol, 4-Phenylamino-anilin, 4- Dimethylamino-anilin, 4-Diethylamino-anilin, 4-[Di(2-hydroxy- ethyl)amino]-anilin, 4-[(2-Methoxyethyl)amino]-anilin, 4-[(3- Hydroxypropyl)amino]-anilin, 1,4-Diamino-2-(2-hydroxyethyl)- benzol, 1,4-Diamino-2-(1-methylethyl)-benzol, 1,3-Bis [(4-aminophenyl)(2-hydroxyethyl)amino]-2-propanol, 1,8-Bis (2,5-diaminophenoxy)-3,6-dioxaoctan, 4-Amino-phenol, 4-Amino- 3-methyl-phenol, 4-Methylamino-phenol, 4-Amino-2-(amino- methyl)-phenol, 4-Amino-2-[(2-hydroxyethyl)-amino]methyl- phenol, 4-Amino-2-(methoxymethyl)-phenol, 4-Amino-2-(2- hydroxyethyl)-phenol, 5-Amino-salicylsSure, 2,5-Diamino- pyridin, 2,4,5,6-Tetraamino-pyrimidin, 2,5,6-Triamino-4- (1H)-pyrimidon, 4,5-Diamino-1-(2-hydroxyethyl)-1H-pyrazol, 4,5- Diamino-1-(1-methylethyl)-1H-pyrazol, 4,5-Diamino-1-[(4-me-thylphenyl)methyl]-1H-pyrazol, 1-[(4-Chlorphenyl)methyl]-4,5-diamino-1H-pyrazol, 4,5-Diamino-1-methyl-1H-pyrazol, 2-Amino- phenol, 2-Amino-6-methyl-phenol, 2-Amino-5-methyl-phenol.

**Kuppler**

N-(3-Dimethylamino-phenyl)-harnstoff, 2,6-Diamino-pyridin, 2- Amino-4-[(2-hydroxyethyl)amino]-anisol, 2,4-Diamino- 1-fluor- 5-methyl-benzol, 2,4-Diamino-1-methoxy-5-methyl-benzol, 2,4- Diamino-1-ethoxy-5-methyl-benzol, 2,4-Diamino-1-(2-hydroxy-ethoxy)-5-methyl-benzol, 2,4-Di[(2-hydroxyethyl)amino]-1,5- dimethoxy-benzol, 2,3-Diamino-6-methoxy-pyridin, 3-Amino-6- methoxy-2-(methylamino)-pyridin, 2,6-Diamino-3,5-dimethoxy- pyridin, 3,5-Diamino-2,6-dimethoxy-pyridin, 1,3-Diamino- benzol, 2,4-Diamino-1-(2-hydroxyethoxy)-benzol, 2,4-Diamino-1,5-di(2-hydroxyethoxy)-benzol, 1-(2-Amino-ethoxy)-2,4-diamino-benzol, 2-Amino-1-(2-hydroxyethoxy)-4- methylamino-benzol, 2,4-Diaminophenoxy-essigsäure, 3-[Di(2- hydroxyethyl)amino]-anilin, 4-Amino-2-di[(2-hydroxyethyl) amino]-1-ethoxy-benzol, 5-Methyl-2-(1-methylethyl)-phenol, 3-[(2-Hydroxyethyl)amino]-anilin, 3-[(2-Aminoethyl)amino]- anilin, 1,3-Di(2,4-diaminophenoxy)-propan, Di(2,4-diamino-phenoxy)-methan, 1,3-Diamino-2,4-dimethoxy-benzol, 2,6-Bis (2-hydroxyethyl)amino-toluol, 4-Hydroxyindol, 3-Dimethylamino- phenol, 3-Diethylamino-phenol, 5-Amino-2-methyl-phenol, 5-Amino-4-fluor-2-methyl-phenol, 5-Amino-4-methoxy-2-methyl- phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2- methylphenol, 3-[(2-Hydroxyethyl)amino]-phenol, 3-[(2-Me-thoxyethyl)amino]-phenol, 5-Amino-2-ethyl-phenol, 2-(4- Amino- 2-hydroxyphenoxy)-ethanol, 5-[(3-Hydroxypropyl)amino]-2- methyl-phenol, 3-[(2,3-Dihydroxypropyl)amino]-2-methyl-phenol, 3-[(2-Hydroxyethyl)amino]-2-methyl-phenol, 2-Amino-3-hydroxy- pyridin, 5-Amino-4-chlor-2-methyl-phenol, 1-Naphthol, 1,5-Di-hydroxy-naphthalin, 1,7-Dihydroxy-naphthalin, 2,3-Dihydroxy-naphthalin, 2,7-Dihydroxy-naphthalin, 2-Methyl-1-naphthol- acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4- methyl-benzol, 1,5-Dichlor-2,4-dihydroxy-benzol, 1,3-Dihy-droxy-2-methyl-benzol, 3,4-Methylendioxy-phenol, 3,4- Methy-lendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino- 3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion.

Die erfindungsgemässe Pulverzubereitung kann zur Herstellung eines Mittels zur Entfärbung von mit oxidativen und gegebenenfalls Nitrofarbstoffen gefärbten Fasern, das Reduktone, wie zum Beispiel Ascorbinsäure oder Isoascorbinsäure beziehungsweise deren Salze oder Ester, beispielsweise 6-O-Palmitoyl-ascorbinsäure, Hydroxypropandial (Trio-sereduktion), 2,3-Dihydroxy-2-cyclopenten-1-on (Reduktinsäure) oder Mischungen dieser Verbindungen, vorzugsweise in einer Menge von 1 bis 50 Gewichtsprozent, insbesondere von 2 bis 10 Gewichtsprozent, enthält, wobei bei Verwendung von Ascorbinsäuresalzen oder Isoascorbinsäure-salzen die freie Säure in situ aus den Salzen, beispielsweise den Alkaliascorbaten oder Erdalkalimetallascorbaten beziehungsweise den Alkaliisoascorbaten oder Erdalkalimetallisoascorbaten, durch Zusatz von Säure erzeugt wird. Dies ist wegen der besseren Löslichkeit der Salze in Wasser insbesondere bei höheren Konzentrationen von Vorteil. Als Ascorbinsäuresalze oder Isoascorbinsäuresalze kommen hierbei insbesondere das Calciumsalz, das Magnesiumsalz und das Natriumsalz der Ascorbinsäure oder Isoascorbinsäure in Betracht.

Bevorzugte Reduktone sind Ascorbinsäure oder Isoascorbinsäure beziehungsweise deren Salze, wobei die Verwendung von Ascorbinsäure besonders bevorzugt ist.

Für die oxidative Erzeugung von Oxidationsfarbstoffen mit Hilfe von Luft oder Sauerstoff in Gegenwart von Enzymen, stehen einstufige oder mehrstufige enzymatische Oxidationssysteme zur Verfügung. Bei den einstufigen Enzymsystemen können aromatische Phenole und Amine in einer Färbemischung unter Sauerstoffzufuhr ohne Peroxidzusatz direkt zum polymeren Farbstoff oxidiert werden. Hierfür sind Phenoloxidasen, vorzugsweise Laccasen, geeignet. Im Gegensatz dazu werden bei den mehrstufigen enzymatischen Oxidationssystemen mehrere Enzyme für die Farbstoffproduktion benötigt.

Für ein mehrstufiges, enzymatisches Oxidationssystem zur Herstellung des Oxidationsfarbstoffes aus den Farbvorstufen kann eine Kombination eines Sauerstoff-Oxidoreductase/Substrat-Systems und einer Peroxidase angewendet werden. Beispiele für ein Sauerstoff-Oxidoreductase/Substrat-System sind folgende:

Glucose-Oxidase (EC 1.1.3.4)/D-Glucose
Alkohol-Oxidase (EC 1.1.3.13)/Ethanol
Pyruvat-Oxidase (EC 1.2.3.3)/Pyruvat
Oxalat-Oxidase (EC 1.2.3.4)/Oxalat
Cholesterin-Oxidase (EC 1.1.3.6)/Cholesterin
Uricase (EC 1.7.3.3)/Harnsäure
Lactat-Oxidase/Milchsäure

Xanthin-Oxidase (EC 1.1.3.22)/Xanthin.

Zur Herstellung eines pulverförmigen Shampoos werden dem erfindungsgemäßen Mittel feste, anionische, kationische, nicht-ionische und/oder amphotere Tenside zugesetzt. Nach Zumischung von Wasser entsteht daraus ein gebrauchsfertiges, gelartiges Shampoo.

Die nachfolgenden Beispiele sollen den Gegenstand der Erfindung näher erläutern.

**Beispiele**

**Beispiel 1:** Tönungspulver mit basischen oder neutralen Farbstoffen

| X g | Farbstoff(e) gemäß Tabelle 1 |
| 0,5 g | Kieselgel |
| 2,0 g | Methylhydroxyethylcellulose (hochverethert) mit einer Viskosität von ca. 10.000 mPa · s (zum Beispiel Tylose MHB 10.000 P der Fa. Hoechst) |

Der Pulvermischung kann Dikaliumhydrogenphosphat oder Zitronensäure zugesetzt sein, so daß der pH-Wert des Fertig-Produkts zwischen 5,5 und 6,5 liegt.

Das Tönungspulver wird in ein verschließbares Behältnis abgepackt. Vor der Anwendung wird es mit warmem Wasser (ca. 30°C) auf 100 g aufgefüllt, ca. 15 Sekunden geschüttelt und auf gebleichte Haare aufgetragen. Nach einer Einwirkungszeit von 20 Minuten bei 40°C wird das Haar mit lauwarmem Wasser gespült und sodann getrocknet. Man erhält die in Tabelle 1 beschriebenen Tönungen.

Tabelle 1

| Nr. | Farbstoff(e) | Farbresultat |
|-----|-------------|--------------|
| 1 A | 0,5 g 4-[Ethyl(2-hydroxyethyl)amino]-1-[(2-hydroxyethyl)-amino]-2-nitro-benzol-hydrochlorid (HC Blue No. 12) | blau-violett |
| 1B | 0,3 g 1-(2-Hydroxyethyl)amino-2-nitro-4-[di(2-hydroxyethyl)amino]-benzol (HC Blue No. 2); <br><br> 0,2 g 1-Amino-5-chlor-4-[2-hydroxyethyl)amino]-2-nitrobenzol | violett |
| 1C | 0,1 g 1-Amino-5-chlor-4-[2-hydroxyethyl)amino]-2-nitrobenzol; <br><br> 0,05 g 1-Amino-5-chlor-4-[(2,3-dihydroxypropyl-(amino]-2-nitrobenzol (HC Red No. 10); <br><br> 0,05 g 5-Chlor-1,4-[di(2,3-dihydroxypropyl)amino]-2-nitrobenzol (HC Red No 11); <br><br> 0,3 g 4-[(3-Hydroxypropyl)amino]-3-nitrophenol | leuchtendes rot |
| 1 D | 0,5 g 1-[(2-Ureidoethyl)amino]-4-nitrobenzol | gelb |
| 1E | 0,3 g 4-[Ethyl(2-hydroxyethyl)amino]-1-[(2-hydroxyethyl)-amino]-2-nitro-benzol-hydrochlorid (HC Blue No. 12); <br><br> 0,2 g 4-[(3-Hydroxypropyl)amino]-3-nitrophenol | beaujolais |
| 1F | 0,1 g 8-Amino-2-brom-5-hydroxy-4-imino-6-[(3-(trimethyl-ammonio)phenyl)amino]-1(4H)-naphthalinon-chlorid (Basic Blue No. 99); <br><br> 0,1 g 2-Hydroxy-1-[(2-methoxyphenyl)azo]-7-(trimethylammonio)-naphthalin-chlorid (Basic Red No. 76); <br><br> 0,3 g 1-[(4-Amino-2-nitrophenyl)azo]-7-(trimethylammonio)-2-naphthol-chlorid (Basic Brown No. 17) | rötliches Braun |

**Beispiel 2:** Tönungspulver mit sauren Farbstoffen

| X g | Farbstoff(e) gemäß Tabelle 2 |
| 1,0 g | Glykolsäure |
| 2,0 g | Methylhydroxyethylcellulose (hochverethert) mit einer Viskosität von ca. 10.000 mPa · s (zum Beispiel Tylose |

MHB 10.000 P der Fa. Hoechst)

0,5 g    Kieselgel

1,0 g    Vanillin

Das Tönungspulver wird in ein verschließbares Behältnis abgepackt. Vor der Anwendung wird es mit warmem Wasser (ca. 30°C) auf 100 g aufgefüllt, ca. 15 Sekunden geschüttelt und auf gebleichte Haare aufgetragen. Nach einer Einwirkungszeit von 20 Minuten bei 40°C wird das Haar mit lauwarmem Wasser gespült und sodann getrocknet. Man erhält die in Tabelle 2 beschriebenen Tönungen.

Tabelle 2

| Nr. | Farbstoff(e) | Farbresultat |
|---|---|---|
| 2A | 0,6 g 4-Hydroxy-3-[(4-sulfo-1-naphthalenyl)azo]-1-naphthalen-sulfonsäure-dinatriumsalz (Acid Red No. 14) | rosarot |
| 2B | 0,02 g 2,4-Dinitro-1-naphthol-7-sulfonsäure-dinatriumsalz (Acid Yellow No. 1); 0,21 g 4-[(2-Hydroxynaphth-1-yl)azo]-benzolsulfonsäure-natriumsalz (Acid orange No. 7); 0,18 g 6-Hydroxy-5-[(4-sulfonaphth-1-yl)azo"-2,4-naphthalin-disulfonsäure-trinatriumsalz (Acid Red No. 18); 0,08 g 1-Hydroxy-4-[(4-methyl-2-sulfophenyl)amino]-9,10-anthra-chinon-natriumsalz (Acid Violet No. 43); 0,11 g 5-Amino-4-hydroxy-6-[(4-nitrophenyl)azo]-3-(phenylazo)-2,7-naphthalin-disulfonsäure-dinatriumsalz (Acid Black No. 1) | braun |
| 2C | 0,6 g 4-Hydroxy-3-[(4-sulfonaphth-1-yl)azo]-1-naphthalin-sulfonsäure-dinatriumsalz (Acid Red No. 14) | dunkelrot |
| 2D | 0,6 g 1-Hydroxy-4-[(4-methyl-2-sulfophenyl)amino]-9,10-anthrachinon-natriumsalz (Acid Violet No. 43) | blau |
| 2E | 0,6 g 4-[(2-Hydroxynaphth-1-yl)azo]-benzolsulfonsäure-natriumsalz (Acid orange No. 7) | orange |
| 2F | 0,6 g 3-Hydroxy-4-[4-sulfonaphth-1-yl)azo]-2,7-naphthalin-disulfonsäure-trinatriumsalz (Acid Red No. 27) | rosarot |

**Beispiel 3:** Haarfärbepulver mit Enzymen

X g           Entwicklersubstanz(en) gemäß Tabelle 3
Y g           Kupplersubstanz(en) gemäß Tabelle 3
Z g           Nitrofarbstoff(e) gemäß Tabelle 3
400 units    Glucose-Oxidase (EC 1.1.3.4)
400 units    Peroxidase (EC 1.11.1.7.)
1,000 g      D-Glucose
0,500 g      Kieselgel
2,000 g      Methylhydroxyethylcellulose (hochverethert) mit einer Viskosität von ca. 10.000 mPa $\cdot$ s (zum Beispiel Tylose MHB 10.000 P der Fa. Hoechst)
0,300 g      Dinatrium-ethylendiaminotetraacetat
0,100 g      Ascorbinsäure
0,280 g      Borsäure

Das Pulver wird mit Natriumhydrogencarbonat vermischt, so daß das nach Wasserzusatz erhaltene Gel einen pH-Wert von 6,5 bis 8 aufweist.

Das Färbepulver wird in ein verschließbares Behältnis abgepackt. Vor der Anwendung wird es mit warmem Wasser (ca. 30°C) auf 100 g aufgefüllt, ca. 15 Sekunden geschüttelt und auf gebleichte Haare aufgetragen. Nach einer Einwirkungszeit von 30 Minuten bei Raumtemperatur (ca. 20 bis 25°C) wird das Haar mit lauwarmem Wasser gespült und sodann getrocknet. Man erhält die in Tabelle 3 beschriebenen Färbungen.

Tabelle 3

| Nr. | Farbstoff(vorstufen) | Farbresultat |
|-----|----------------------|--------------|
| 3A | 0,55 g 1,4-Diamino-2-methyl-benzolsulfat; <br> 0,70 g 2-Amino-4-[(2-hydroxyethyl)amino]-anisol-sulfat | tiefblau |
| 3B | 0,62 g 1,4-Diamino-2-(hydroxyethyl)-benzolsulfat; <br> 0,67 g 2,4-Diamino-1-(2-hydroxyethoxy)-benzol-dihydrochlorid | tiefblau |
| 3C | 0,31 g 4-Amino-3-methyl-phenol <br> 0,70 g 2-Amino-4-[(2-hydroxyethyl)amino]-anisol-sulfat | purpurrot |
| 3D | 0,310 g 4-Amino-3-methyl-phenol <br> 0,080 g 5-Amino-2-methyl-phenol <br> 0,520 g 2-Amino-4-[(2-hydroxyethyl)amino]-anisol <br> 0,075 g 2-Amino-6-chloro-4-nitro-phenol | kupferfarben |

**Beispiel 4:** Mittel zur Färbung und Entfärbung von Fasern

**4a Haarfärbemittel**

| | |
|---|---|
| X g | Entwicklersubstanz(en) gemäß Tabelle 4 |
| Y g | Kupplersubstanz(en) gemäß Tabelle 4 |
| Z g | Nitrofarbstoffe) gemäß Tabelle 4 |
| 0,30 g | Dinatrium-ethylendiaminotetraacetat |
| 0,40 g | Natriumsulfit |
| 10,00 g | Natriumlaurylethersulfat (28prozentige wäßrige Lösung) |
| 10,00 g | Isopropanol |
| 9,10 g | Ammoniak (25%-ige wäßrige Lösung) |
| ad 100,00 g | Wasser, vollentsalzt |

5g der vorstehenden Farbträgermasse werden mit 5 g einer 6prozentigen Wasserstoffperoxidlösung vermischt. Das erhaltene gebrauchsfertige Oxidationshaarfärbemittel wird auf die Haare aufgetragen und mit einem Pinsel gleichmäßig verteilt. Nach einer Einwirkungszeit von 30 Minuten bei 40°C wird das Haar mit lauwarmem Wasser gespült und sodann getrocknet.

**4b Entfärbegel**

| | |
|---|---|
| 6,00 g | Ascorbinsäure |
| 2,00 g | Methylhydroxyethylcellulose (hochverethert) mit einer Viskosität von ca. 10.000 mPa · s (zum Beispiel Tylose MHB 10.000 P der Fa. Hoechst) |
| 0,5 g | Kieselgel |

Das Entfärbegel-Pulver wird in einem verschließbaren Behältnis abgepackt. Vor der Anwendung wird das Pulver mit Wasser auf 100 g aufgefüllt, ca. 15 Sekunden geschüttelt und auf die gefärbten Haare aufgetragen. Das gefärbte Haar wird mit den vorstehenden Entfärbemitteln jeweils 30 Minuten lang bei 40°C behandelt, danach gründlich mit Wasser und einem Shampoo gewaschen und sodann getrocknet. Das gefärbte Haar erhält annähernd seine Ausgangsfarbe zurück.

Das Ergebnis dieser Entfärbehandlung ist in Tabelle 4 zusammengefaßt.

Tabelle 4

| Nr. | Entwickler/Kuppler- Kombination | Farbton nach der Färbung | Farbmeßwerte L a b | Entfärbung [%] |
|---|---|---|---|---|
| 4A | 2,2 g 1,4-Diamino-2-methyl-ben-zolsulfat; <br><br> 1,23 g 5-Amino-2-methylphenol | tiefviolett | unbehandelte Haare: 34,41; +7,27; +13,78 Nach dem Färben: 20,04; +7,55; +0,08 nach 1 x Entfärben (60 min., 40°C): 31,85; +9,28; +14,54 | 83 |
| 4B | 1,2 g 4,5-Diamino-1-(2-hydroxyethyl)-1H-pyrazol; <br><br> 0,62 g 5-Amino-2-methylphenol | intensiv orange-rot | unbehandelte Haare: 34,41; +7,27; +13,78 Nach dem Färben: 27,66; +23,98;+15,06 nach 1 x Entfärben (60 min., 40°C): 33,83; +10,92;+15,08 | 78 |
| 4C | 1,25 g 1,4-Diamino-(2-hydroxethyl)-benzol-sulfat; <br><br> 1,20 g 2,4-Diamino-1-(2-hydroxyehtoxy)-benzol-dihydrochlorid: | blau-schwarz | unbehandelte Haare: 34,41; +7,27; +13,78 Nach dem Färben: 19,76; +0,70; -2,15 nach 1 x Entfärben (60 min., 40°C): 32,31; +10,31;+12,98 | 83 |
| 4D | 1,92 g 4-Amino-3-methylphenol; <br><br> 0,32 g 1-Naphthol; <br><br> 1,38 g 5-Amino-2-methylphenol; <br><br> 0,61 g 2-Amino-4[(2-hydroxyethyl)amino]-anisol sulfat | rotbraun | unbehandelte Haare: 34,41; +7,27; +13,78 Nach dem Färben: 28,18; +15,19;+11,12 nach 1 x Entfärben (60 min., 40°C): 34,65; +9,63; +14,76 | 75 |
| 4E | 0,55 g 1,4-Diamino-2-methylbenzol-sulfat; <br><br> 0,5 g 2-Methyl-1-naphthol-acetat; <br><br> 0,31 g 5-Amino-2-methylphenol | tiefviolett | unbehandelte Haare: 34,41; +7,27; +13,78 Nach dem Färben: 20,74; +7,91; -0,53 nach 1 x Entfärben (60 min., 40°C) 35,20; +9,05; +14,40 | 90 |
| 4F | 0,83 g 1,4-Diamino-2-methyl-ben-zolsulfat; <br><br> 0,42 g 2-Amino-4-(2-hydroxyethyl)-aminoanisol-sulfat; <br><br> 0,46 g 4-Amino-3-methylphenol | dunkelbraun | unbehandelte Haare: 34,41 ; +7,27; +13,78 Nach dem Färben: 21,22; +4,66; +3,90 nach 1 x Entfärben (60 min., 40°C): 33,87; + 7,53; +13,97 | 96 |

**Beispiel 5:** Pulvershampoo A

10,0 g   Lauramidopropyl Betaine (zum Beispiel Rewoteric AM B 12 P der Firma WITCO)
2,0 g   Methylhydroxyethylcellulose (hochverethert) mit einer Viskosität von ca. 10.000 mPa • s (zum Beispiel Tylose MHB 10.000 P der Firma Hoechst)
0,5 g   Kieselgel

Das Pulvershampoo wird in ein verschließbares Behältnis abgepackt. Vor der Anwendung wird es mit Wasser auf 100 g aufgefüllt, ca. 15 Sekunden geschüttelt und wie ein übliches Shampoo verwendet.

**Beispiel 6:** Pulvershampoo B

10,0 g   Disodium Ricinoleamido Mea-sulfosuccinate (zum Beispiel Rewoderm S 1333 Pulver der Firma WITCO)
2,0 g   Methylhydroxyethylcellulose (hochverethert) mit einer Viskosität von ca. 10.000 mPa • s (zum Beispiel Tylose MHB 10.000 P der Firma Hoechst)
0,5 g   Kieselgel
0,3 g   Zitronensäure

Das Pulvershampoo wird in ein verschließbares Behältnis abgepackt. Vor der Anwendung wird es mit Wasser auf 100 g aufgefüllt, ca. 15 Sekunden geschüttelt und wie ein übliches Shampoo verwendet.

**Beispiel 7:** Pulvershampoo C

10,0 g     Disodium Lauramido Mea-(zum Beispiel Rewopol SB L 203 P)
2,0 g      Methylhydroxyethylcellulose (hochverethert) mit einer Viskosität von ca. 10.000 mPa • s (zum Beispiel Tylose MHB 10.000 P der Fa. Hoechst)
0,5 g      Kieselgel
0,3 g      Zitronensäure

Das Pulvershampoo wird in ein verschließbares Behältnis abgepackt. Vor der Anwendung wird es mit Wasser auf 100 g aufgefüllt, ca. 15 Sekunden geschüttelt und wie ein übliches Shampoo verwendet.

Die in den vorliegenden Beispielen ermittelten L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Der Wert D gibt die Farbdifferenz an, die zwischen den unbehandelten und den gefärbten bzw. entfärbten Strähnchen besteht. Er wird folgendermaßen bestimmt:

$$D = \sqrt{(L_i\text{-}L_o)^2 + (a_i\text{-}a_o)^2 + (b_i\text{-}b_o)^2}$$

wobei $L_0$, $a_o$ und $b_o$ die Farbmesswerte von unbehandeltem Haar und $L_i$, $a_i$ und $b_i$ die Werte des behandelten Haares darstellen.

Die Entfärberate in Prozent wurde folgendermaßen ermittelt:

Entfärbe-% = [1 - (D nach Entfärbung/D nach Färbung)] x 100.

Alle in der vorliegenden Anmeldung genannten Prozentangaben stellen, soweit nicht anders angegeben, Gewichtsprozente dar. Die für die Enzyme in Klammern angegebenen Klassifizierungen erfolgten gemäss der "Classification on Nomenclature and Classification of Enzymes (1984)". Die Enzymkonzentrationen werden in der vorliegenden Anmeldung in "units" - der von der Internationalen Union für Biochemie als Standardeinheit für alle Enzyme vorgeschlagenen Internationalen Einheit - angegeben.

**Patentansprüche**

1. Pulverförmiges Mittel zum Herstellen von kosmetisch verwendbaren Gelen, dadurch gekennzeichnet, daß es eine Methylhydroxyalkylcellulose, welche in einer 2prozentigen wäßrigen Lösung eine Viskosität von 7.000 bis 12.000 mPa • s aufweist in einer Menge von 0,5 bis 50 Gewichtsprozent enthält.

2. Pulverförmiges Mittel nach Anspruch 1, dadurch gekennzeichnet, daß es Methylhydroxyethylcellulose enthält.

3. Pulverförmiges Mittel nach Anspruch 2, dadurch gekennzeichnet, daß es als Methylhydroxyethylcellulose eine hochveretherte Methylhydroxyethylcellulose enthält, die in einer 2prozentigen wäßrigen Lösung eine Viskosität von etwa 10.000 mPa • s aufweist.

4. Pulverförmiges Mittel nach den Ansprüchen 1 bis 3, dadurch gekennzeichnet, daß es die Methylhydroxyalkylcellulose in einer Menge von 1 bis 10 Gewichtsprozent enthält.

5. Pulverförmiges Mittel nach den Ansprüchen 1 bis 4, dadurch gekennzeichnet, daß es zusätzlich Füllstoffe und/oder Trocknungsmittel enthält.

6. Pulverförmiges Mittel nach den Ansprüchen 1 bis 5, dadurch gekennzeichnet, daß es die in einem Tönungsgel, einem Haarfärbegel, einem Reduktone enthaltenden Entfärbegel oder einem Shampoo für kosmetische Anwendungen üblichen Inhaltsstoffe enthält.

7. Verfahren zum Herstellen eines gebrauchsfertigen, kosmetisch verwendbaren Gels, dadurch gekennzeichnet, daß es nach Zugabe eines Lösungsmittels, insbesondere von Wasser bei einer Temperatur zwischen etwa 15 bis 40'C, zu dem pulverförmigen Mittel der Ansprüche 1 bis 6 durch kurzes Umschütteln erzeugt wird.